# FASCICULE DE BREVET EUROPEEN

(11) **EP 2 378 863 B1**
(45) Date de publication et mention de la délivrance du brevet: **08.02.2017**
(21) Numéro de dépôt: 09797039.6
(22) Date de dépôt: 18.12.2009
(51) Int. Cl.: A01K 11/00, A61B 10/02

(54) **DISPOSITIF DE PRÉLÉVEMENT D'UN ÉCHANTILLON DE TISSU D'UN ANIMAL**
VORRICHTUNG ZUR ENTNAHME VON GEWEBEPROBEN VON EINEM TIER
DEVICE FOR SAMPLING TISSUE FROM AN ANIMAL

(30) Priorité: 19.12.2008 FR 0807262
(43) Date de publication de la demande: 26.10.2011
(73) Titulaire: Allflex Europe, 35500 Vitre (FR)
(72) Inventeur: HILPERT, Jean-Jacques, 35500 Vitre (FR); DECALUWE, Johan, 53000 Laval (FR)
(74) Mandataire: Vidon Brevets & Stratégie
(86) Numéro de dépôt international: PCT/EP2009/067590
(87) Numéro de publication internationale: WO 2010/070129

(56) Documents cités:
- EP-A- 1 504 722
- EP-A- 1 772 104

## Description

### 1. Domaine de l'invention

Le domaine de l'invention est celui du contrôle et/ou de l'identification des animaux.

Plus précisément, l'invention concerne le prélèvement de tissu d'un animal, permettant notamment de conserver des cellules portant des caractéristiques biologiques ou biochimiques de l'animal, par exemple pour identifier ultérieurement l'animal ou détecter des maladies de l'animal. De tels prélèvements sont par exemple effectués lors de la mise en place d'un dispositif de marquage, encore appelé marque.

L'invention permet notamment le prélèvement de tissu sur des bovins, des ovins, des porcins, des caprins, des volatiles, des poissons, ou plus généralement sur toute espèce animale.

### 2. Techniques de l'art antérieur

Afin d'améliorer le suivi du cheptel et garantir l'origine et la traçabilité des animaux destinés notamment à la consommation, il est de plus en plus souvent procédé à un ou plusieurs prélèvements d'échantillons de tissu sur ces animaux au cours de leur existence.

Un premier prélèvement peut être effectué sur l'animal à sa naissance, par exemple lors de la pose d'une marque d'identification de l'animal. D'autres prélèvements peuvent être effectués par la suite, par exemple pour détecter des maladies ou encore pour certifier l'identité de l'animal, par comparaison des séquences d'ADN, ...

Une fois collecté, l'échantillon de tissu de l'animal peut être transmis directement à un laboratoire pour analyse. L'échantillon peut également être stocké à titre conservatoire sur plusieurs mois à plusieurs années, en le plongeant dans un agent conservateur par exemple.

Pour faciliter le prélèvement d'un échantillon de tissu sur un animal, il est connu d'utiliser un emporte-pièce métallique pouvant être monté sur un des mors, ou mâchoire, d'une pince de prélèvement, par exemple de façon amovible. Cet emporte-pièce est classiquement placé à l'extrémité d'un pointeau solidarisé à une mâchoire.

Diverses formes d'arêtes coupantes de cet emporte-pièce ont été proposées pour perforer la peau de l'animal et détacher l'échantillon : forme sensiblement circulaire ou forme plus complexe, à contact continu ou en dents de scie.

On a également pensé à prélever l'échantillon lors de la pose sur l'animal d'une marque d'identification (par exemple au niveau d'une oreille) reconstituée par emboîtement ou encliquetage d'une partie mâle dans une partie femelle.

On connaît ainsi une technique pour laquelle la tige saillante de la partie mâle présente un passage cylindrique creux permettant le passage d'un emporte-pièce, encore appelé poinçon.

Ainsi, lors de l'emboîtement des parties mâle et femelle, le pointeau de la pince de prélèvement pousse l'emporte-pièce qui perfore alors la peau de l'animal avec son arête coupante le long d'une ligne de contact circonférentielle, tout en guidant la tige saillante de la partie mâle dans la partie femelle. L'emporte-pièce est retiré après avoir mis la marque en place, et l'échantillon de tissu prélevé demeuré à l'intérieur de l'emporte-pièce peut être récupéré.

Un inconvénient de ces techniques de prélèvement associées ou non à une opération de marquage de l'animal, est que l'échantillon peut se détacher inopinément de l'emporte-pièce et être contaminé.

Pour répondre à ce problème, il a été proposé un emporte-pièce amovible se fixant sur un premier mors, ou mâchoire, de l'outil de prélèvement. Cet emporte-pièce s'emmanche au moins partiellement dans un récipient en forme de tube fixé à un deuxième mors de l'outil lors de l'actionnement de l'outil, et se détache du premier mors, ce qui permet de le retirer avec le récipient, auquel il peut servir de couvercle hermétique. Grâce à cette technique de prélèvement, décrite dans le document EP 1 014 861, l'échantillon est directement inséré dans le récipient, limitant ainsi le risque de contamination de l'échantillon.

Il est clair cependant, qu'un inconvénient commun aux techniques décrites précédemment réside dans le fait que l'utilisateur est contraint de désinfecter l'emporte-pièce et l'outil après chaque prélèvement pour éviter tout contamination de l'animal suivant, ce qui est fastidieux.

Un autre inconvénient commun à ces techniques connues est souvent la difficulté pour l'utilisateur de monter l'emporte-pièce sur l'outil, du fait des dimensions de ce dernier ou, lorsque les conditions climatiques l'imposent, du port de gants, ... qui gêne l'utilisateur.

Encore un inconvénient de ces techniques connues est que l'emporte-pièce doit être manipulé pour extraire l'échantillon de tissu resté dans la partie tubulaire centrale de l'emporte-pièce après découpe, cette dernière opération pouvant se révéler malaisée et/ou complexe, et/ou présente un risque de coupure pour l'utilisateur.

Il existe donc un besoin pour une nouvelle technique de prélèvement de tissu d'un animal ne présentant pas l'ensemble de ces inconvénients de l'art antérieur.

### 3. Objectifs de l'invention

L'invention a notamment pour objectif de pallier ces différents inconvénients de l'état de l'art.

Plus précisément, l'invention a pour objectif de fournir un dispositif de prélèvement d'un échantillon de tissu d'un animal qui soit fiable et exempt de risque pour l'animal et l'utilisateur.

Dans au moins un mode de réalisation particulier de l'invention, un objectif de l'invention est de fournir un dispositif de prélèvement qui ne nécessite pas une décontamination de l'outil sur lequel il est monté, entre deux prélèvements.

Un autre objectif de l'invention est notamment, dans au moins un mode de réalisation particulier, de permettre à un utilisateur de procéder à un ou plusieurs prélèvements successifs sans devoir enfiler des gants pour se protéger d'une éventuelle contamination.

Un autre objectif de l'invention est de fournir un dispositif de prélèvement pouvant être utilisé avec un outil de prélèvement et/ou de marquage existant.

Plus précisément, dans un mode de réalisation particulier, un objectif de l'invention est de pouvoir utiliser un tel dispositif de prélèvement avec un outil de marquage déjà commercialisé, comme la pince Total Tagger (marque déposée) par exemple.

Encore un autre objectif de l'invention est de fournir un dispositif de prélèvement dont le montage et le démontage sont simples.

### 4. Exposé de l'invention

Ces objectifs, ainsi que d'autres qui apparaîtront par la suite, sont atteints à l'aide d'un dispositif de prélèvement de tissu d'un animal selon la revendication 1.

L'invention propose ainsi une nouvelle approche du prélèvement d'un échantillon de tissu sur un animal, particulièrement simple et rapide pour l'utilisateur. Elle simplifie notamment l'opération de montage et de démontage sur le pointeau de l'outil et permet de ne plus avoir à systématiquement décontaminer l'outil entre deux prélèvements successifs, ce qui est doublement avantageux.

Selon un aspect particulier de l'invention, lesdits moyens de prélèvement comprennent des moyens de solidarisation entre ledit élément de coupe et ledit élément de montage et de protection, formés de façon que ledit élément de coupe puisse être désolidarisé dudit élément de protection après ledit prélèvement.

Ainsi, l'élément de coupe peut être aisément détaché de l'élément de montage et de protection après prélèvement.

Préférentiellement, après prélèvement, ledit élément de montage et de protection étant apte à coopérer avec des moyens de montage temporaire prévus sur ledit outil comprenant au moins un élément appartenant au groupe comprenant : élément de clippage, élément de vissage, élément de montage à force, celui-ci reste monté sur ledit outil.

Ainsi, l'élément de montage et de protection étant retiré avec l'outil après le prélèvement, sans qu'il soit nécessaire de procéder à une opération supplémentaire sur l'animal, on évite les risques de contamination de l'animal par l'utilisateur ou vice-versa.

Selon un aspect particulier de l'invention, ledit élément de montage et de protection amovible est configuré de sorte à pouvoir être démonté dudit outil en agissant sur lesdits moyens de montage temporaire et/ou sur ledit outil.

Ce démontage peut notamment être obtenu par éjection de l'élément de montage et de protection.

Dans au moins un mode de réalisation avantageux de l'invention, au moins deux portions distinctes dudit élément de montage et de protection sont solidarisées audit pointeau, de façon à augmenter la rigidité dudit élément de montage et de protection.

Ainsi, l'élément de montage et de protection peut présenter des portions rigides et des portions plus souples, par exemple pour faciliter le montage et/ou réduire sa masse et donc son coût, tout en soutenant l'élément de coupe de façon convenable, et conserver des qualités de perforation satisfaisantes au dispositif.

De façon avantageuse, une première desdites portions est disposée sensiblement au voisinage de la base dudit élément formant pointeau et une deuxième desdites portions est disposée à l'extrémité la plus fine dudit élément formant pointeau.

Ainsi, l'élément de montage et de protection est monté efficacement sur le pointeau.

Avantageusement, ledit élément de montage et de protection est réalisé en matière biodégradable.

Ainsi, une fois le prélèvement effectué, l'utilisateur peut se débarrasser simplement de l'élément de montage et de protection, par exemple en le jetant au sol sur place, tout en préservant l'environnement.

Dans au moins un mode de réalisation de l'invention, ledit élément de montage et de protection est en matériau au moins partiellement déformable.

De cette façon, le montage sur l'outil de l'élément de montage et de protection, et son retrait, sont facilités.

Avantageusement, ledit élément de montage et de protection est enduit d'une substance antiseptique.

Ainsi, on simplifie le nettoyage de la plaie provoquée par le prélèvement sur l'animal et/ou on réduit les risques de contamination par l'outil, l'utilisateur ou l'animal, de l'élément de montage et de protection.

Dans au moins un mode de réalisation particulier de l'invention, ledit élément de coupe et ledit élément de montage et de protection présentent une forme de révolution et sont d'axes confondus.

Notamment, dans au moins un mode particulier de l'invention, l'élément de coupe et /ou l'élément de montage et de protection peuvent présenter une forme sensiblement conique.

Selon un aspect particulier de l'invention, une des extrémités dudit élément de montage et de protection présente des moyens de centrage sur ledit dispositif de prélèvement et/ou de marquage.

Avantageusement, ladite extrémité dudit élément de montage et de protection présente une ouverture essentiellement tronconique apte à coopérer avec la base dudit ledit élément formant pointeau.

De façon avantageuse, un dispositif de prélèvement tel que décrit ci-dessus comprend des moyens de stockage aptes à recevoir ledit élément de coupe et à le désolidariser dudit élément de montage et de protection.

Ainsi, la manipulation de l'élément de coupe est aisée et l'échantillon est stocké sur place avec l'élément de coupe, ce qui réduit le risque de souiller l'échantillon.

De manière astucieuse, ledit élément de coupe forme des moyens de fermeture d'une ouverture formée dans lesdits moyens de stockage.

L'élément de coupe peut ainsi servir de bouchon hermétique aux moyens de stockage sur lesquels il peut être monté, par exemple, par emboîtement, clippage ou vissage.

L'invention concerne également un élément de montage et de protection constitutif d'un dispositif de prélèvement de tissu d'un animal tel que présenté ci-dessus.

### 5. Liste des figures

D'autres caractéristiques et avantages de l'invention apparaîtront plus clairement à la lecture de la description suivante de deux modes de réalisation particuliers, donnés à titre de simples exemples illustratifs et non limitatifs, et des dessins annexés, parmi lesquels :
- la figure 1 illustre les moyens de prélèvement d'un dispositif de prélèvement selon un mode de réalisation de l'invention, montés sur le pointeau d'un outil de prélèvement en vu de procéder à un prélèvement ;
- la figure 2 est une vue en coupe de l'élément de montage et de protection du dispositif de prélèvement présenté sur la figure 1 ;
- la figure 3 illustre un tube de stockage recevant l'élément de coupe du dispositif présenté sur la figure 1, après l'avoir aidé à se désolidariser de l'élément de montage et de protection ;
- les figures 4A et 4B illustrent un exemple de montage, avant prélèvement, et de démontage, après prélèvement, de l'élément de montage et de protection du dispositif présenté sur la figure 1, sur l'outil, à l'aide de moyens de montage temporaire avantageux.

### 6. Description détaillée d'un mode de réalisation de l'invention

### 6.1. Rappel du principe de l'invention

Le principe général de l'invention repose sur la mise en oeuvre inédite de moyens de prélèvement dotés d'une protection active de l'outil de prélèvement et/ou de marquage sur lequel ces moyens sont destinés à être montés. De façon indirecte, l'invention permet également d'éviter la contamination de l'élément de coupe des moyens de prélèvement, mais aussi de l'animal et de l'utilisateur.

L'invention propose ainsi d'intégrer aux moyens de prélèvement un élément de montage et de protection amovible, dans le prolongement de l'élément de coupe, qui s'étend avantageusement sur au moins une partie substantielle du pointeau de l'outil de prélèvement et/ou de marquage, pour éviter un contact avec l'animal et/ou l'échantillon prélevé, et donc éviter à l'utilisateur la contrainte de devoir décontaminer l'outil après chaque prélèvement.

Il est proposé, dans au moins un mode de réalisation de l'invention, un élément de montage et de protection s'adaptant sur le pointeau d'un outil de prélèvement et/ou de marquage de type connu. Dans au moins un autre mode de réalisation de l'invention, l'élément de montage et de protection est réalisé en matière biodégradable, pour être jeté après utilisation.

### 6.2. Exemple d'un mode de réalisation de l'invention

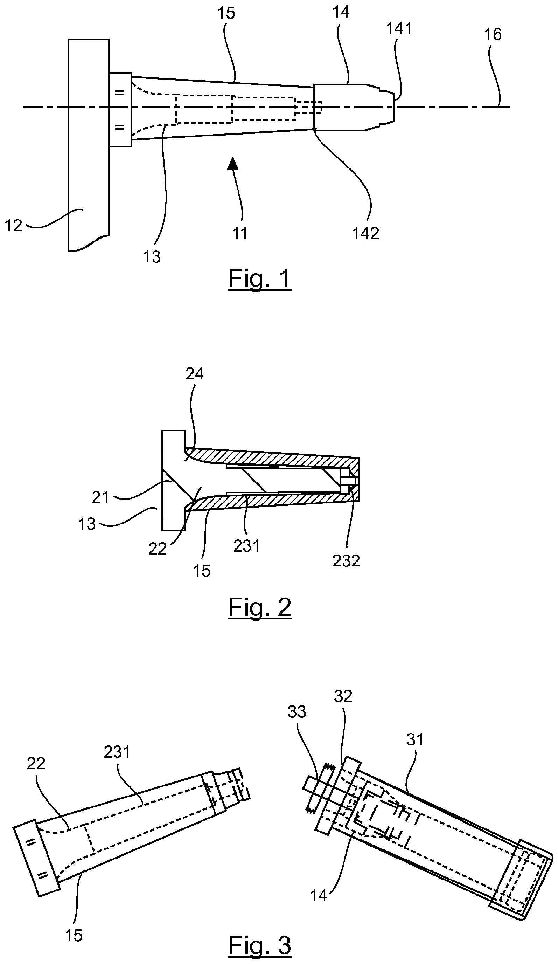

On présente en relation avec la figure 1 un exemple de mode de réalisation de l'invention dans lequel le dispositif de prélèvement de tissu d'un animal selon l'invention est utilisé avec une pince de prélèvement de type connu.

Comme illustré sur la figure 1, les moyens de prélèvement de tissu selon l'invention 11 ont été monté par l'utilisateur sur la pince 12 au voisinage d'un élément formant pointeau 13, pour effectuer un prélèvement unique.

Comme déjà évoqué, le montage de ce dispositif se révélant particulièrement simple et l'utilisation de ce dispositif permettant de s'affranchir de l'étape de décontamination de l'outil entre deux prélèvements, on constate avantageusement une accélération significative de la cadence de prélèvement des échantillons sur un troupeau.

Les moyens de prélèvement de tissu 11 comprend un élément de coupe 14 (« emporte-pièce »), présentant une forme de révolution, et un élément de montage et de protection 15 («manchon») disposé dans le prolongement de l'élément de coupe 14, avec lequel il est aligné suivant un même axe 16. Les longueurs respectives de l'élément de coupe 14 et de l'élément de montage et de protection représentent environ 70 pourcents et 30 pourcents de la longueur totale du dispositif de prélèvement dans ce mode de réalisation de l'invention.

L'élément de coupe 14 présente une arête coupante 141 de forme circulaire régulière, permettant de perforer la peau 13 de l'animal lorsque la pince 12 est actionnée, et des moyens de solidarisation 142 avec l'élément de montage et de protection 15 au niveau de sa base, représenté sur la figure 1 dans un premier état dans lequel l'élément de montage et de protection 15 est solidarisé à l'élément de coupe 14.

Dans ce mode de réalisation, l'élément de montage et de protection 15, ou chemise de montage et de protection, enveloppe l'élément formant pointeau sensiblement sur toute sa longueur. Il présente en particulier une forme extérieure sensiblement tronconique, pour épouser la forme générale de l'élément formant pointeau 13. Dans des variantes de ce mode de réalisation de l'invention, d'autres formes de l'élément de montage et de protection peuvent également être envisagé, celles-ci pouvant avantageusement être choisies parmi les formes de révolution.

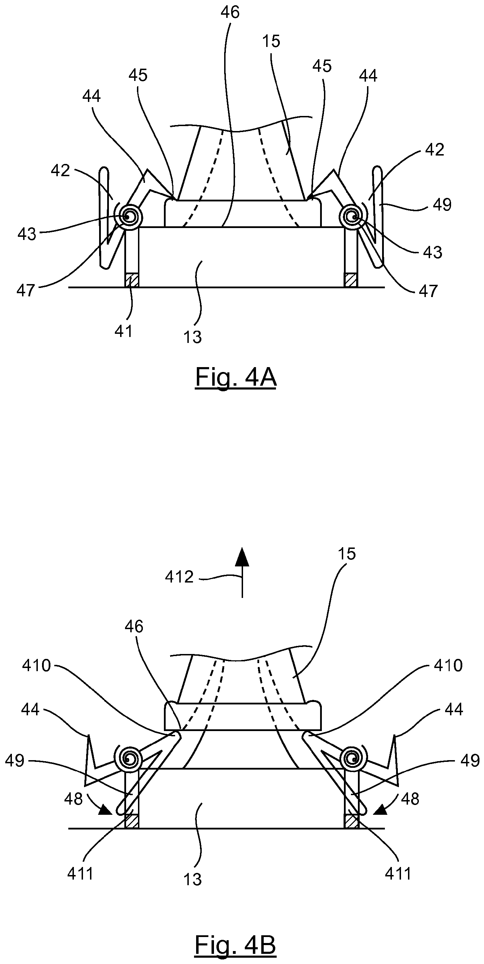

Pour faciliter le montage et la retenue de l'élément de montage et de protection sur l'outil de prélèvement (par exemple une pince), on a monté, dans ce mode de réalisation de l'invention, au voisinage du pointeau de ce dernier, un équipement supplémentaire illustré sur la figure 4A. Cet équipement présente une base 41 sensiblement en forme d'anneau sur laquelle sont montés deux leviers 42 articulés autour de leurs axes respectifs 43, dont un premier bras 44 forme une griffe prévue pour s'écarter du pointeau 13 dans une première étape, puis dans une deuxième étape venir enserrer un renflement 45 à la base 46 de l'élément de montage et de protection 15 sous l'action d'un ressort de rappel 47. Ces deux étapes sont mises en oeuvre lorsque l'élément de montage et de protection 15 est progressivement poussé dans la direction de la base de l'élément formant pointeau 13 pour être mis en place sur l'outil de prélèvement.

Dans des variantes de ce mode de réalisation de l'invention, il peut également être envisagé de mettre en oeuvre d'autres moyens de montage temporaire sur outil que l'équipement illustré sur la figure 4A, tels que par exemple un ou plusieurs éléments de clippage et/ou de vissage et/ou de montage à force.

Il convient de noter que l'élément de montage et de protection 15 est en matériau au moins partiellement déformable pour faciliter sa pose sur la base de l'élément formant pointeau 13 et/ou l'assemblage de l'élément de montage et de protection 15 avec la base de l'élément de coupe 14.

Comme on peut le voir sur la figure 2, dans une représentation en coupe, l'élément de montage et de protection 15 comprend un évidemment interne 21 le traversant de part en part. Cet évidemment 21 présente, au voisinage de la base 46 de l'élément de montage et de protection 15, une ouverture évasée 22 sensiblement en forme de pavillon permettant de l'assembler et de le centrer sur la base 24 de l'élément formant pointeau 15, suivie d'un tronçon tronconique intermédiaire 231 et d'un tronçon tubulaire d'extrémité 232, de diamètres décroissants lorsqu'on s'éloigne de l'ouverture 22, pour épouser la forme du pointeau. Le tronçon 232 est solidarisé à l'extrémité la plus fine de l'élément formant pointeau 13, de façon à renforcer et/ou augmenter la rigidité de l'élément de montage et de protection 15.

Dans des variantes de ce mode de réalisation, il peut également être prévu de réduire l'épaisseur de la paroi de l'élément de montage et de protection au niveau des tronçons intermédiaires, et/ou de ne recouvrir qu'une partie du pourtour du pointeau avec l'élément de montage et de protection.

De façon à pouvoir être jeté sur place par l'utilisateur après utilisation, l'élément de montage et de protection 15 est réalisé dans ce mode de réalisation en matière biodégradable, par exemple à partir d'extraits de maïs ou de riz. Une substance antiseptique est enduite sur la surface extérieure de l'élément de montage et de protection 15, pour nettoyer la plaie de l'animal lorsque l'élément de montage et de protection 15 pénètre avec l'élément formant pointeau 13 qu'il protège, au travers de l'orifice circulaire laissé par l'élément de coupe 14 lors du prélèvement d'un échantillon de tissu de l'animal.

Dans ce mode de réalisation de l'invention, le dispositif de prélèvement comprend un tube de stockage pour recevoir et aider à désolidariser l'élément de coupe 14. Ce tube de stockage se fixe sur le mors de la pince ne présentant pas d'élément formant pointeau.

Lorsque la pince est actionnée, l'élément de coupe 14 est poussé par l'élément de montage et de protection 15 et l'élément formant pointeau 13 à l'intérieur du tube de stockage, après avoir découpé l'échantillon de tissu.

Lorsque la pince est relâchée, le tube de stockage 31 retient l'élément de coupe 14, dont la base se désolidarise de l'élément de montage et de protection 15, ce dernier restant monté autour de l'élément formant pointeau 13, comme on peut le constater sur la figure 3, qui illustre un deuxième état du dispositif de prélèvement, postérieur au prélèvement.

Le tube de stockage 31 contenant l'échantillon peut alors être retiré de la pince, puis refermé à l'aide, par exemple, d'un bouchon hermétique 33, emmanché, ou vissé dans son ouverture 32. L'élément de coupe 14 peut également servir de bouchon hermétique.

L'échantillon ainsi stocké peut être transmis à un laboratoire pour analyse, ou être conservé pour une durée plus ou moins longue, de l'ordre de quelques jours à quelques années grâce à l'effet d'un agent conservateur présent ou injecté par la suite dans le tube de stockage.

On rappelle que le stockage d'échantillons de tissu d'un animal permet notamment d'identifier ultérieurement l'animal ou de détecter des maladies chez cet animal, au vu d'un examen microscopique ou d'une extraction d'une séquence d'ADN de cellules de l'échantillon par exemple.

La figure 4B illustre finalement un exemple de démontage, après prélèvement, de l'élément de montage et de protection du dispositif présenté sur la figure 1.

Comme on peut le voir sur la figure 4B, l'élément de montage et de protection 15 peut être retiré de l'outil après utilisation, en pressant le deuxième bras 49 des leviers 42 dans la direction des flèches 48, entraînant un mouvement de rotation des premier 44 et deuxième 49 bras de chaque levier autour de leur axe 43 respectif. La portion coudée 410 du deuxième bras 49 de chacun des leviers 42, après avoir traversé une rainure de passage 411, peut venir pousser sur la base 46 de l'élément de montage et de protection et ainsi éjecter l'élément de montage et de protection 15 de l'outil dans la direction de la flèche 412.

### 6.2. Autres caractéristiques et avantages de l'invention

Dans au moins un autre mode de réalisation de l'invention, il peut également être envisagé :
- de prélever un échantillon de tissu lors la mise en place d'une marque auriculaire sur un animal, avec un dispositif de prélèvement selon l'invention coopérant avec un outil de marquage ;
- une arête coupante de l'élément de coupe en dents de scie ;

## Revendications

1. Dispositif de prélèvement de tissu d'un animal, apte à coopérer avec un outil de prélèvement et/ou de marquage (12), comprenant des moyens de prélèvement (11) destinés à être placés sur un élément formant pointeau (13) dudit outil,
lesdits moyens de prélèvement (11) comprenant un élément de coupe (14) apte à prélever un échantillon desdits tissus ;
**caractérisé en ce que** lesdits moyens de prélèvement (11) comprennent également un élément amovible de protection (15) dudit pointeau, apte à recouvrir au moins une partie substantielle dudit pointeau, comprenant des moyens de montage sur ledit pointeau,
ledit élément de coupe (14) étant disposé dans le prolongement dudit élément de protection (15).

2. Dispositif de prélèvement selon la revendication 1, **caractérisé en ce que** lesdits moyens de prélèvement (11) comprennent des moyens de solidarisation réversibles entre ledit élément de coupe (14) et ledit élément de protection (15).

3. Dispositif de prélèvement selon la revendication 1, **caractérisé en ce que** lesdits moyens de montage sur ledit pointeau sont aptes à coopérer avec des moyens de montage temporaire complémentaires prévus sur ledit outil, et comprennent au moins un élément appartenant au groupe comprenant : un élément de clippage, un élément de vissage, un élément de montage à force.

4. Dispositif de prélèvement selon l'un quelconque des revendications 1 à 3, **caractérisé en ce qu'**au moins deux portions distinctes dudit élément de protection (15) sont solidarisées audit pointeau, de façon à augmenter la rigidité dudit élément de protection (15).

5. Dispositif de prélèvement selon la revendication 4, **caractérisé en ce qu'**une première desdites portions est disposée sensiblement au voisinage de la base dudit pointeau et une deuxième desdites portions est disposée à l'extrémité la plus fine dudit pointeau.

6. Dispositif de prélèvement selon l'un quelconque des revendications 1 à 5, **caractérisé en ce que** ledit élément de protection (15) est réalisé en matière biodégradable.

7. Dispositif de prélèvement selon l'un quelconque des revendications 1 à 6, **caractérisé en ce que** ledit élément de protection (15) est enduit d'une substance antiseptique.

8. Dispositif de prélèvement selon l'un quelconque des revendications 1 à 7, **caractérisé en ce que** ledit élément de protection (15) est en matériau au moins partiellement déformable.

9. Dispositif de prélèvement selon l'un quelconque des revendications 1 à 8, **caractérisé en ce que** ledit élément de coupe (14) et ledit élément de protection (15) présentent une forme de révolution et sont d'axes confondus.

10. Dispositif de prélèvement selon l'un quelconque des revendications 1 à 9, **caractérisé en ce qu'**une des extrémités dudit élément de protection (15) présente des moyens de centrage sur ledit outil de prélèvement et/ou de marquage.

11. Dispositif de prélèvement selon la revendication 10, **caractérisé en ce que** ladite extrémité dudit élément de protection (15) présente une ouverture essentiellement tronconique apte à coopérer avec la base dudit pointeau.

12. Dispositif de prélèvement selon l'une quelconque des revendications 1 à 11, **caractérisé en ce qu'**il comprend des moyens de stockage aptes à recevoir ledit élément de coupe (14) et à le désolidariser dudit élément de protection (15).

13. Dispositif de prélèvement selon l'une quelconque des revendications 1 à 12, **caractérisé en ce que** ledit élément de coupe (14) forme des moyens de fermeture d'une ouverture formée dans lesdits moyens de stockage.

14. Elément de protection (15) destiné à coopérer avec des moyens de prélèvement (11) d'un dispositif de prélèvement de tissu d'un animal apte à coopérer avec un outil de prélèvement et/ou de marquage (12) dudit animal, lesdits moyens de prélèvement étant destinés à être placés sur un élément formant pointeau (13) dudit outil, et comprenant en outre un élément de coupe (14) apte à prélever un échantillon desdits tissus,
**caractérisé en ce que** ledit élément de protection (15) est un élément amovible de protection , apte à recouvrir au moins une partie substantielle dudit pointeau,
ledit élément de protection (15) comprenant des moyens de montage configurés pour monter ledit élément de protection sur ledit pointeau et disposer ledit élément de protection dans le prolongement dudit élément de coupe (14).

## Patentansprüche

1. Vorrichtung zur Entnahme von Gewebe eines Tiers, die zum Zusammenwirken mit einem Werkzeug zur Entnahme und/oder Markierung (12) geeignet ist, wobei die Vorrichtung Mittel zur Entnahme (11) aufweist, die dazu vorgesehen sind, auf ein eine Nadel bildenden Element (13) des Werkzeugs angeordnet zu werden,
wobei die Mittel zur Entnahme (11) ein Schneideelement (14) aufweisen, das zum Entnehmen einer Probe der Gewebe geeignet ist;
**dadurch gekennzeichnet, dass** die Mittel zur Entnahme (11) weiter ein entfernbares Schutzelement (15) der Nadel aufweisen, das geeignet ist, wenigstens einen wesentlichen Teil der Nadel abzudecken, und das Mittel zur Montage auf der Nadel aufweist,
wobei das Schneideelement (14) in der Verlängerung des Schutzelementes (15) angeordnet ist.

2. Vorrichtung zur Entnahme nach Anspruch 1, **dadurch gekennzeichnet, dass** die Mittel zur Entnahme (11) lösbare Verbindungsmittel zwischen dem Schneideelement (14) und dem Schutzelement (15) aufweisen.

3. Vorrichtung zur Entnahme nach Anspruch 1, **dadurch gekennzeichnet, dass** die Mittel zur Montage auf der Nadel geeignet sind, mit auf dem Werkzeug vorgesehenen komplementären temporären Befestigungsmitteln zusammenzuwirken, die wenigstens ein Element ausgewählt aus der folgenden Gruppe umfassen: ein Clipselement, ein Schraubelement, ein Kraftbefestigungselement.

4. Vorrichtung zur Entnahme nach einem der Ansprüche 1 bis 3, **dadurch gekennzeichnet, dass** wenigstens zwei unterschiedliche Abschnitte des Schutzelements (15) mit der Nadel verbunden sind, um die Steifigkeit des Schutzelementes (15) zu erhöhen.

5. Vorrichtung zur Entnahme nach Anspruch 4, **dadurch gekennzeichnet, dass** ein erster der Abschnitte im Wesentlichen in Umgebung der Basis der Nadel angeordnet ist, und ein zweiter der Abschnitte an dem dünnsten Ende der Nadel angeordnet ist.

6. Vorrichtung zur Entnahme nach einem der Ansprüche 1 bis 5, **dadurch gekennzeichnet, dass** das Schutzelement (15) aus biologisch abbaubarem Material realisiert ist.

7. Vorrichtung zur Entnahme nach einem der Ansprüche 1 bis 6, **dadurch gekennzeichnet, dass** das Schutzelement (15) von einer antiseptischen Substanz überzogen ist.

8. Vorrichtung zur Entnahme nach einem der Ansprüche 1 bis 7, **dadurch gekennzeichnet, dass** das Schutzelement (15) aus einem wenigstens teilweise verformbaren Material besteht.

9. Vorrichtung zur Entnahme nach einem der Ansprüche 1 bis 8, **dadurch gekennzeichnet, dass** das Schneideelement (14) und das Schutzelement (15) eine Rotationsform mit zusammenfallenden Achsen aufweisen.

10. Vorrichtung zur Entnahme nach einem der Ansprüche 1 bis 9, **dadurch gekennzeichnet, dass** eines der Enden des Schutzelementes (15) Mittel zum Zentrieren auf dem Werkzeug zur Entnahme und/oder der Markierung aufweist.

11. Vorrichtung zur Entnahme nach Anspruch 10, **dadurch gekennzeichnet, dass** das Ende des Schutzelementes (15) eine im Wesentlichen kegelstumpfförmige Öffnung aufweist, die zum Zusammenwirken mit der Basis der Nadel geeignet ist.

12. Vorrichtung zur Entnahme nach einem der Ansprüche 1 bis 11, **dadurch gekennzeichnet, dass** sie Mittel zum Lagern aufweist, die geeignet sind, das Schneideelement (14) aufzunehmen und es von dem Schutzelement (15) zu trennen.

13. Vorrichtung zur Entnahme nach einem der Ansprüche 1 bis 12, **dadurch gekennzeichnet, dass** das Schneideelement (14) Mittel zum Schließen einer in den Mitteln zum Lagern gebildeten Öffnung bildet.

14. Schutzelement (15), das vorgesehen ist, mit Mitteln zur Entnahme (11) einer Vorrichtung zur Entnahme von Gewebe eines Tiers, die zum Zusammenwirken mit einem Werkzeug zur Entnahme und/oder zur Markierung (12) des Tieres geeignet sind, zusammenzuwirken, wobei die Mittel zur Entnahme vorgesehen sind, auf einem eine Nadel bildenden Element (13) des Werkzeugs angeordnet zu werden und weiter ein Schneideelement (14) aufweisen, das zum Entnehmen einer Probe des Gewebes geeignet ist,
**dadurch gekennzeichnet, dass** das Schutzelement (15) ein lösbares Schutzelement ist, das geeignet ist, wenigstens einen wesentlichen Teil der Nadel abzudecken, wobei das Schutzelement (15) Mittel zur Montage aufweist, die eingerichtet sind, das Schutzelement auf der Nadel zu montieren und das Schutzelement in der Verlängerung des Schneideelements (14) anzuordnen.

## Claims

1. Animal tissue sampling device configured to cooperate with a sampling and/or tagging tool (12), including sampling means (11) intended to be placed on a rod-forming element (13) of said tool,
said sampling means (11) comprising a cutting element (14) configured to collect a sample of said tissues, **characterized in that** said sampling means (11) also comprises a removable protection element (15) for protection of said rod, designed to cover at least a substantial portion of said rod, including means for assembly on said rod,
said cutting element (14) being arranged in the extension of said protection element.

2. Sampling device according to claim 1, **characterized in that** said sampling means (11) include reversible securing means between said cutting element (14) and said protection element (15).

3. Sampling device according to claim 1, **characterized in that** said means for assembly on said rod are configured to cooperate with complementary temporary assembly means provided on said tool, and include at least one element belonging to the group including: a clipping element, a screwing element, or a force-fitted assembly element.

4. Sampling device according to any one of claims 1 to 3, **characterized in that** at least two distinct portions of said protection element (15) are secured to said rod, so as to increase the rigidity of said protection element (15).

5. Sampling device according to claim 4, **characterized in that** a first of said portions is arranged substantially in the vicinity of the base of said rod and a second of said portions is arranged at the finest end of said rod.

6. Sampling device according to any one of claims 1 to 5, **characterized in that** said protection element (15) is made of a biodegradable material.

7. Sampling device according to any one of claims 1 to 6, **characterized in that** said protection element (15) is coated with an antiseptic substance.

8. Sampling device according to any one of claims 1 to 7, **characterized in that** said protection element (15) is made of an at least partially deformable material.

9. Sampling device according to any one of claims 1 to 8, **characterized in that** said cutting element (14) and said protection element (15) have a revolutional shape and coincident axes.

10. Sampling device according to any one of claims 1 to 9, **characterized in that** one of the ends of said protection element (15) has centering means on said sampling and/or tagging tool.

11. Sampling device according to claim 10, **characterized in that** said end of said protection element (15) has an essentially frusto-conical opening configured to cooperate with the base of said rod.

12. Sampling device according to any one of claims 1 to 11, **characterized in that** it includes storage means configured to receive said cutting element (14) and to detach it from said protection element (15).

13. Sampling device according to any one of claims 1 to 12, **characterized in that** said cutting element (14) forms means for closing an opening formed in said storage means.

14. Protection element (15) designed to cooperate with sampling means (11) of an animal tissue sampling device configured to cooperate with a tool for sampling and/or tagging (12) said animal,
said sampling means being intended to be placed on a rod-forming element (13) of said tool, and also including a cutting element (14) configured to collect a sample of said tissue,
**characterized in that** said protection element (15) is a removable protection element, configured to cover at least a substantial portion of said rod,
said protection element (15) including means for assembly designed to assemble said protection element (15) on said rod and arranged said protection element in the extension of said cutting element (14).
